Europäisches Patentamt

European Patent Office

Office européen des brevets

(19)

(11) Veröffentlichungsnummer : **0 014 333**
**B1**

# (12) EUROPÄISCHE PATENTSCHRIFT

(45) Veröffentlichungstag der Patentschrift :
**11.11.81**

(21) Anmeldenummer : **80100164.5**

(22) Anmeldetag : **14.01.80**

(51) Int. Cl.³ : **C 07 G   7/00, A 61 K 37/02//**
**B01D15/00**

(54) **Verfahren zur Herstellung von Fibrinogen, einem die Gerinnungsfaktoren II, VII, IX und X enthaltenden Prothrombinkomplex, Antithrombin III und einer Lösung von lagerstabilen Serumproteinen.**

(30) Priorität : **20.01.79 DE 2902158**

(43) Veröffentlichungstag der Anmeldung :
**20.08.80 (Patentblatt 80/17)**

(45) Bekanntmachung des Hinweises auf die Patenterteilung : **11.11.81 Patentblatt 81/45**

(84) Benannte Vertragsstaaten :
**AT BE CH DE FR GB IT NL SE**

(56) Entgegenhaltungen :
**DE - A - 1 617 335**
**DE - A - 2 029 455**
**DE - A1 - 2 459 291**
**DE - A1 - 2 725 711**
**FR - A1 - 2 362 633**

(73) Patentinhaber : **Biotest-Serum-Institut GmbH**
**Flughafenstrasse 4**
**D-6000 Frankfurt-Niederrad (DE)**

(72) Erfinder : **Kotitschke, Ronald, Dr.phil.nat. Dipl.-Chem.**
**Forsthausstrasse 4**
**D-6071 Götzenhain (DE)**
Erfinder : **Stephan, Wolfgang, Dr.phil.nat. Dipl.-Chem.**
**Philipp-Holzmannstrasse 84**
**D-6072 Dreieich (DE)**

(74) Vertreter : **Beil, Walter, Dr. et al**
**BEIL, WOLFF & BEIL Rechtsanwälte Adelonstrasse 58**
**D-6230 Frankfurt am Main 80 (DE)**

Verfahren zur Herstellung von Fibrinogen, einem die Gerinnungsfaktoren II, VII, IX and X enthaltenden Prothrombinkomplex, Antithrombin III und einer Lösung von lagerstabilen Serumproteinen

Die Erfindung betrifft ein Verfahren zur Herstellung von Fibrinogen, einem die Gerinnungsfaktoren II, VII, IX und X enthaltenden Prothrombinkomplex, der Antithrombin III enthalten kann, Antithrombin III und einer Lösung von lagerstabilen Serumproteinen aus einem mit Citrat stabilisierten Blutplasma, das dadurch gekennzeichnet ist, daß man aus dem Plasma durch Adsorption an kolloidaler Kieselsäure mit einer spezifischen Oberfläche von 50 bis 400 m²/g, die in einer Konzentration von 50 bis 400 mg je g Plasmaprotein angewendet wird, Fibrinogen isoliert; anschließend entweder

a) zunächst durch Ultrafiltration oder Dialyse Citrat- und Calciumionen entfernt und dann aus der Proteinlösung über Proteine adsorbierenden Anionenaustauschern oder Tricalciumphosphat die Gerinnungsfaktoren II, VII, IX und X und Antithrombin III adsorbiert oder

b) die Gerinnungsfaktoren II, VII, IX und X vor der Ultrafiltration oder Dialyse adsorbiert, wobei Antithrombin III nicht gleichzeitig adsorbiert wird, und Antithrombin III erst nach der Entfernung der Citrat- und Calciumionen durch Ultrafiltration oder Dialyse an den genannten Adsorbentien adsorbiert; und sodann aus der verbleibenden Plasmaflüssigkeit weitere unstabile Proteine durch eine erneute Adsorption an kolloidaler Kieselsäure entfernt und eine Lösung von lagerstabilen Serumproteinen gewinnt.

Im erfindungsgemäßen Verfahren werden aus einem mit Citrat stabilisierten Blutplasma unter Vermeidung des Gerinnungsvorganges drei gerinnungsaktive, hepatitissichere und therapeutisch anwendbare Eiweißpräparate und ein gerinnungsinaktives, hepatitissicheres Serum bzw. eine Lösung lagerstabiler Serumproteine erhalten. Die drei gerinnungsaktiven Präparate sind:

1. Fibrinogen

2. Prothrombinkomplex (Gerinnungsfaktoren II, VII, IX und X, gegebenenfalls AT III-haltig)

3. Antithrombin III (AT III).

Die gerinnungsfaktoren II, VII, IX und X werden bekanntlich auch als PPSB-Faktoren bezeichnet gemäß folgender Bedeutung:

P = Prothrombin (Faktor II)

P = Proconvertin (Faktor VII)

S = Stuart-Prower-Faktor (Faktor X)

B = antihämophiles Globulin B (Faktor IX).

In der DE-OS 24 59 291 ist ein Verfahren zur Herstellung eines antihämophiles Globulin A enthaltenden Konzentrats neben einem Prothrombin-Komplex und einer Lösung von lagerstabilen Serumproteinen beschrieben, bei dem als Ausgangsmaterial ein gefrorenes Ionenaustauscherplasma verwendet wird, das bei Temperaturen von 2 bis 8 °C wieder aufgetaut wird und aus dem die überstehende Plasmaflüssigkeit von dem Faktor-VIII-enthaltenden Proteinniederschlag abgetrennt und dieses Kryopräzipitat in an sich bekannter Weise zu einem Faktor-VIII-Konzentrat aufgearbeitet wird, während die Plasmaflüssigkeit durch Adsorption über handelsüblichem Tricalciumphosphat von den Faktoren II, VII, IX und X befreit wird, und anschließend aus der Plasmaflüssigkeit über kolloidaler Kieselsäure mit einer spezifischen Oberfläche von 50 bis 400 m²/g die noch im Plasma vorliegenden Gerinnungsfaktoren und andere unstabile Proteine adsorbiert werden und eine Lösung von lagerstabilen Serumproteinen zurückbleibt.

In der gleichlaufenden deutschen Patentanmeldung P 29 03 131.0, die einen Zusatz zu P 25 59 291.4 darstellt, wird eine Abänderung des vorstehend beschriebenen Verfahrens vorgeschlagen, bei der als Ausgangsmaterial ein übliches Citratplasma verwendet und nach dem Auftauen und Abtrennen des Kyzropräzipitates der Überstand mit Anionenaustauscherharzen auf Basis Polystyrol/Divinylbenzol/quaternäres Amin und Kationenaustauscherharzen auf Basis Polystyrol/Sulfonat behandelt wird. Bei diesem Verfahren wird also Citratplasma in Ionenaustauscherplasma verwandelt und aus diesem Ionenaustauscherplasma läßt sich neben antihämophilem Globulin A, dem Prothrombinkomplex und lagerstabilen Serumproteinen noch Fibrinogen herstellen.

Überraschenderweise wurde nun gefunden, daß man zur Herstellung von Fibrinogen, einem die Gerinnungsfaktoren II, VII, IX und X enthaltenden Prothrombinkomplex und einer Lösung von lagerstabilen Serumproteinen nicht nur Ionenaustauscherplasma — wie es in den genannten Anmeldungen verwendet wird —, sondern auch ein über kolloidaler Kieselsäure mit einer spezifischen Oberfläche von 50 bis 400 m²/g adsorbiertes und dialysiertes oder ultrafiltriertes Citratplasma verwenden kann.

Der im erfindungsgemäßen Verfahren herstellbare Prothrombinkomplex enthält angereichert Antithrombin III, das sich von den Faktoren II, VII, IX und X trennen läßt und als zusätzliches Produkt gewonnen werden kann. Nach der schrittweisen Elimination der Gerinnungsfaktoren aus dem Citratplasma erhält man die Lösung lagerstabiler Serumproteine.

Die Herstellung von Antithrombin III neben dem Prothrombinkomplex ist auch möglich, ohne daß dabei zunächst ein Antithrombin III-haltiger Prothrombinkomplex hergestellt werden muß.

In den DE-PSn 16 17 319 und 16 17 335 wurde die Verwendung von kolloidaler Kieselsäure in einer Konzentration von 250 bis 500 mg je Gramm Gesamtprotein bei Temperaturen von 20 bis 50 °C zur Entfernung von Lipoproteinen und Fibrinogen beschrieben. Ziel der zitierten Patentschriften war die Herstellung stabiler und steriler Serumproteinlösungen.

Überraschenderweise wurde nun gefunden, daß bei Verwendung geringer Konzentrationen an kolloidaler Kieselsäure bei der Adsorption von Citratplasma bei Temperaturen von 1 bis 37 °C nahezu spezifisch Fibrinogen an die kolloidale Kieselsäure gebunden wird, während die Gerinnungsfaktoren II, VII, IX und X in dem von der kolloidalen Kieselsäure befreiten «Rest-Citratplasma» erhalten bleiben. Diese Trennung erfolgt besonders gut bei Temperaturen von 2 bis 6 °C, insbesondere 4 °C.

Durch die Entfernung des Fibrinogens aus dem Citratplasma wird es möglich, dieses «Citratplasma» zu dialysieren oder zu ultrafiltrieren, um somit die Citrat- und Calciumionen aus diesem «Plasma» zu entfernen. Die Dialyse oder Ultrafiltration eines fibrinogenhaltigen Citratplasmas ist nicht möglich, weil es durch die Entfernung des Citratstabilisators auch ohne die Gegenwart von Calciumionen zu Fibrinogenfällungen kommt, die die Membranen verstopfen und die technische Durchführung einer Dialyse oder Ultrafiltration prinzipiell unmöglich machen.

Die nachstehende Tabelle I zeigt die Abhängigkeit der Konzentration des Fibrinogens und der Faktoren II, VII, IX und X sowie des Faktors XII von der bei der Adsorption angewandten Konzentration der kolloidalen Kieselsäure («Aerosil AE 380», geschütztes Warenzeichen der Fa. Degussa) unter den Bedingungen des erfindungsgemäßen Verfahrens und den in den DE-PSn 16 17 335 und 16 17 319 genannten Bedingungen. Als Ausgangsmaterial a wurde ein unbehandelter Citratplasmapool eingesetzt; als Ausgangsmaterial b wurde ein mit ß-Propiolacton und UV-Bestrahlung behandelter Citratplasmapool verwendet. Durch die Behandlung von Plasma mit ß-Propiolacton und UV-Bestrahlung wird zwar die Aktivität der Gerinnungsfaktoren vermindert (R. Kotitschke und W. Stephan: Struktur und Funktion des Fibrinogens, Schattauer-Verlag, Stuttgart-New York 1976, S. 22-228); diese Behandlung mit ß-Propiolacton und UV-Bestrahlung dient jedoch der Sterilisation, um womöglich im Plasma vorhandene Viren zu inaktivieren und insbesondere die Hepatitisfreiheit der Proteinfaktoren zu gewährleisten.

Tabelle I

Abhängigkeit der Konzentration der Gerinnungsfaktoren von der bei der Adsorption angewandten Konzentration der kolloidalen Kieselsäure

| Kieselsäure mg/g Protein | Aktivität der Faktoren in % der Norm | | | | | Fibrinogen mg/100 ml |
|---|---|---|---|---|---|---|
| | II | VII | IX | X | XII | |
| 0 (Ausgangsmaterial a*) | 100 | 100 | 100 | 100 | 100 | 320 |
| 250 bei +4 °C | 95 | 92 | 98 | 95 | 0 | 0 |
| 500 bei 45 °C | 0 | 0 | 0 | 0 | 0 | 0 |
| 0 (Ausgangsmaterial b**) | 50 | 60 | 60 | 55 | 55 | 200 |
| 250 bei +4 °C | 45 | 55 | 55 | 50 | 0 | 0 |
| 500 bei +45 °C | 0 | 0 | 0 | 0 | 0 | 0 |

\* Human-Citratplasmapool
\*\* mit ß-Propiolacton und UV-Bestrahlung behandelter Human-Citratplasmapool

Die Bezeichnung «Restcitratplasma» soll deutlich machen, daß die Bezeichnung dieser Proteinlösung zu Nomenklatur-Schwierigkeiten führt, da üblicherweise unter Plasma eine Proteinlösung verstanden wird, die noch Fibrinogen in gerinnbarer Form enthält. Fibrinogenfreie Proteinlösungen werden üblicherweise als Serum bezeichnet, so daß man das «Restcitratplasma» auch als «Restcitratserum» bezeichnen könnte. Unter einem Serum versteht man jedoch die Proteinlösung, die aus einem Plasma oder aus Blut entsteht, nachdem der Gerinnungsvorgang abgelaufen ist. In der erfindungsgemäß mit kolloidaler Kieselsäure adsorbierten Proteinlösung sind nun aber die Gerinnungsfaktoren II, VII, IX und X noch als Proenzyme bzw. in aktivierbarer Form enthalten und somit auch noch für einen Gerinnungsablauf zu verwenden, während das Fibrinogen nicht mehr vorhanden ist.

Werden nun durch Dialyse oder Ultrafiltration das ursprünglich für die Stabilisierung des Blutes erforderliche Citrat und gleichzeitig auch die Calciumionen aus der mit kolloidaler Kieselsäure adsorbierten Proteinlösung entfernt, so erhält man eine stabilisatorfreie Proteinlösung, die noch gerinnungsaktive Gerinnungsfaktoren enthält.

Die nachstehende Tabelle II zeigt die Zusammensetzung des Citratplasmas und der erfindungsgemäß nach Adsorption an kolloidaler Kieselsäure und nach Ultrafiltration erhaltenen Lösungen im Hinblick auf den Gehalt an Protein, Fibrinogen, Citrat und Calciumionen.

Tabelle II

| Gehalt an: | Protein g/100 ml | Fibrinogen mg/100 ml | Citrat mäq/l | $Ca^{++}$ mäq/l |
|---|---|---|---|---|
| Citratplasma | 5,65 | 320 | 60 | 4,5 |
| Nach $SiO_2$-Adsorption | 5,29 | 0 | 60 | 4,4 |
| Nach Ultrafiltration | 5,20 | 0 | 0,5 | 0,30 |

Das unterschiedliche Verhalten eines normalen Citratplasma im Vergleich zu dem mit kolloidaler Kieselsäure adsorbierten und ultrafiltrierten Citratplasma wird auch aus der folgenden Tabelle III deutlich. Mit 1,5 g DEAE-Sephadex A-50 pro 1 l Plasma werden etwa 8 % der Proteine aus dem Citratplasma adsorbiert, während aus dem an Kieselsäure adsorbierten und ultrafiltrierten Plasma unter diesen Bedingungen etwa 23 % der Plasmaproteine adsorbiert werden.

Tabelle III
Proteinkonzentration in g/100 ml

| | Citratplasma normal | Citratplasma an SiO$_2$ adsorbiert und ultrafiltriert |
|---|---|---|
| Ausgang | 5,65 | 5,20 |
| DEAE-Sephadex A-50 (1,5 g/l) | 5,20 | 4,00 |
| DEAE-Sephadex A-50 (0,5 g/l) | 5,40 | 4,70 |

Aus der nach der SiO$_2$-Adsorption und Ultrafiltration oder Dialyse erhaltenen Lösung lassen sich die Gerinnungsfaktoren II, VII, IX und X sowie Antithrombin III an Tricalciumphosphat oder überraschenderweise auch an Proteine adsorbierenden Anionenaustauschern, insbesondere Diethylaminoethylgruppen tragende, quervernetzte Dextrane oder Cellulose, z.B. an «DEAE-Sephadex» oder «DEAE-Cellulose», adsorbieren.

Die nachstehende Tabelle IV zeigt in einem Vergleich die unterschiedliche Adsorbierbarkeit von Antithrombin III aus normalen und mit kolloidaler Kieselsäure adsorbiertem und ultrafiltriertem Citratplasma. Als kolloidale Kieselsäure wurde «Aerosil 380» (AE 380) verwendet.

Tabelle IV
Adsorbierbarkeit von AT III

| | AT III in Citratplasma normal (IU*/ml) | AT III in Citratplasma AE 380, ultrafiltriert (IU*/ml) |
|---|---|---|
| Ausgangsmaterial | 19,1 | 19,1 |
| nach Adsorption mit « DEAE-Sephadex A 50 » | 17,5 | 10,3 |

* IU = Inhibitor-Einheiten (Inhibitor Units) gemäß der Bestimmung mit einem Peptidsubstrat (Tos-Gly-Pro-Arg-pNA).

Das Antithrombin III läßt sich zusammen mit den PPSB-Faktoren mit Hilfe citrathaltiger Lösungen vom Tricalciumphosphat oder von den Proteine adsorbierenden Anionenaustauschern eluieren. Die Bedeutung dieses Befundes liegt darin, daß bisherigen PPSB-Präparaten ohne Antithrombin III ein Thromboserisiko angelastet wurde (S. Chandra und M. Wickerhauser: Preparation of a Nonthrombogenic Prothrombin Complex Concentrate. Thrombos. Haemostat. VI Int. Cong., Schattauer Verlag, Stuttgart-New York, 1977, S. 219).

Daher wurde in den letzten Jahren die Forderung aufgestellt, PPSB-Präparate mit entsprechenden Tests auf ihr Thromboserisiko hin zu überprüfen, wobei als Maß für das Thromboserisiko die TGt$_{50}$-Zeit eingeführt wurde. Mit der TGt$_{50}$-Zeit wird die Inkubationszeit bestimmt, die erforderlich ist, um eine Fibrinogengerinnungszeit von 50 Sekunden zu erreichen. Mit ihrer Hilfe wird also die Bildung von Thrombin bestimmt. (J. D. Cash, G. Sas, R. G. Dalton u. R. Owens: Thrombogenicity of Factor IX Concentrates. Workshop on inhibitors of factors VIII and IX, Wien 26. — 27.1.1976, S. 112-117).

PPSB-Präparate ohne Antithrombin III besitzen TGt$_{50}$-Zeiten unter 20 Minuten, wenn sie unter Verwendung von «DEAE-Sephadex» aus Citratplasma hergestellt werden, während die entsprechenden Antithrombin-III-haltigen PPSB-Präparate aus mit Aerosil adsorbiertem und ultrafiltriertem Citratplasma TGt$_{50}$-Zeiten von über 5 Stunden besitzen.

Bei den in der Literatur beschriebenen, bekannten Verfahren (J. Heystek, H. G. J. Brummelhuis, H. W. Krijnen: Contributions to the Optical Use of Human Blood. II. The Large-Scale Preparation of Prothrombin Complex. A Comparison between 2 Methods Using the Anion Exchangers DEAE-Cellulose DE 52 and DEAE-Sephadex A 50. Vox. Sang. 25: Nr. 2 S. 113-123 (1973)) der Herstellung von PPSB aus Citratplasma unter Verwendung von «DEAE-Sephadex A 50» bzw. «DEAE-Cellulose» wurden die PPSB-Faktoren mit Citrat/NaCl-Pufferlösung vom Absorbens eluiert. Überraschenderweise wurde nun gefunden, daß — wie in Tabelle IV gezeigt — Antithrombin III mit an Proteine adsorbierenden Anionenaustauscherharzen, z.B. «DEAE-Sephadex A 50» oder «DEAE-Cellulose», adsorbiert wird, wenn die Citrationenkonzentration im Citratplasma stark erniedrigt wird. Die Tabelle V zeigt, wie entscheidend die Citratkonzentration für die Antithrombin-III-Adsorbierbarkeit an die genannten Adsorbentien ist.

Tabelle V

Abhängigkeit der AT III-Adsorbierbarkeit an «DEAE-Sephadex A 50» von der Citratkonzentration in einem mit «Aerosil 380» adsorbierten und anschließend dialysierten oder ultrafiltrierten Citratplasma.

| Citrat mäq/l | AT III in IU/ml | |
| --- | --- | --- |
| | Vor DEAE-Sephadex-Adsorption | Nach DEAE-Sephadex-Adsorption |
| 63 | 17,5 | 17,0 |
| 10 | 17,5 | 16,8 |
| 2,5 | 17,5 | 15,9 |
| 0,5 | 17,5 | 10,5 |

Aus den Daten der Tabelle V wird auch der überraschende Befund verständlich, daß man z.B. gemäß Abb. I ein Antithrombin III-Präparat dadurch gewinnen kann, daß man mit kolloidaler Kieselsäure adsorbiertes und anschließend ultrafiltriertes oder dialysiertes Citratplasma mit Proteine adsorbierenden Anionenaustauschern, z.B. «DEAE-Sephadex A 50» adsorbiert und das adsorbierte Antithrombin III anschließend durch niedrige Citratkonzentrationen, z.B. 0,01M Citrat in physiologischer NaCl-Lösung, eluiert. Unter diesen Bedingungen werden die PPSB-Faktoren nicht vom «DEAE-Sephadex» eluiert. In Abhängigkeit von der Citratkonzentration des Elutionsmittels wird Antithrombin III mehr oder weniger eluiert. Da es aus den vorstehend aufgeführten Gründen wünschenswert ist, Antithrombin III in PPSB zu haben (Minderung des Thromboserisikos), wird man bei der Elution des Antithrombin III von den Proteine adsorbierenden Anionenaustauschern zur Herstellung eines Antithrombin III-Konzentrates eine Citratkonzentration wählen, bei der nicht das gesamte Antithrombin III eluiert wird, sondern noch etwas Antithrombin III an den Proteine adsorbierenden Anionenaustauschern verbleibt und erst mit den PPSB-Faktoren zusammen vollständig eluiert wird.

Das an die kolloidale Kieselsäure adsorbierte Fibrinogen läßt sich mit 5 bis 20 % Alkalihalogenide enthaltenden Pufferlösungen, z.B. citrathaltigen 10 bis 20 %igen Kochsalzlösungen, bei pH-Werten von 8,0 bis 10,0 eluieren. Gerinnbares Fibrinogen läßt sich durch Ultrafiltration dieser Lösungen und anschließender Alkoholfraktionierung gewinnen.

Durch erneute Adsorption mit kolloidaler Kieselsäure gemäß den in den obengenannten Patentschriften angegebenen Bedingungen oder auch davon abweichenden Adsorptionsbedingungen bezüglich der $SiO_2$-Konzentration, Temperatur und Zeit, erhält man unter Adsorption von Lipiden aus der über Tricalciumphosphat oder Proteine adsorbierenden Anionenaustauschern adsorbierten Proteinlösung eine Lösung lagerstabiler Serumproteine, die frei von Gerinnungsfaktoren oder Metaboliten der Gerinnungsfaktoren ist.

Das erfindungsgemäße Verfahren zur Herstellung der Gerinnungsfaktoren II, VII, IX und X, Fibrinogen und Antithrombin III läßt sich auch realisieren, wenn aus dem Citratplasma (gegebenenfalls nach Entfernung von Kryopräzipitat) zunächst die Gerinnungsfaktoren II, VII, IX und X (PPSB) durch Adsorption mit Proteine adsorbierenden Anionenaustauschern entfernt werden und dieses Plasma anschließend mit kolloidaler Kieselsäure zur Adsorption des Fibrinogens gemischt wird. Bei diesem Vorgehen wirkt sich allerdings die häufig bereits ablaufende Fibrinolyse gegebenenfalls nachteilig auf die Fibrinogengewinnung aus. Das von der kolloidalen Kieselsäure getrennte «Citratplasma» dient dann nach seiner Ultrafiltration als Ausgangsmaterial zur Gewinnung des Antithrombin III und der Lösung der Serumproteine, wobei das Antithrombin III durch Adsorption an Tricalciumphosphat oder Proteine adsorbierenden Anionenaustauschern und anschließende Elution gewonnen wird. Diese Variante ist in Abb. II dargestellt. Weitere ähnliche Varianten ergeben sich aus den Abb. IIa und IIb.

Die Abb. III und IV zeigen weitere Ausführungsformen, bei denen die Reihenfolge der Adsorptions- und Ultrafiltrationsschritte verändert wurde. Allen Ausführungsformen des erfindungsgemäßen Verfahrens ist gemeinsam, daß ein Citratplasma durch Adsorption mit kolloidaler Kieselsäure und danach erfolgende Ultrafiltration oder Dialyse in die günstigste weiter zu verarbeitende Form gebracht wird, die unter Vermeidung der in der Proteinfraktionierung üblichen Fraktioniermethoden, die alle einen ungünstigen Einfluß auf die native Struktur der Proteine haben, die gleichzeitige Herstellung mehrerer gerinnungsaktiver Produkte und einer gerinnungsinerten Lösung von Serumproteinen erlaubt.

Nach dem erfindungsgemäßen Verfahren wird als Ausgangsmaterial ein mit Citrat stabilisiertes Plasma verwendet. Aus diesem Plasma kann in an sich bekannter Weise zunächst durch Auftauen bei Temperaturen nahe + 4 °C ein Kryopräzipitat gewonnen werden, das dann weiter zu Konzentraten von antihämophilem Globulin A aufgearbeitet werden kann. Mit dem von Kryopräzipitat befreiten Plasma läßt sich dann das erfindungsgemäße Verfahren durchführen. Es ist aber auch möglich, ein Citratplasma, das nicht von Kryopräzipitat befreit wurde, zu verwenden. Das verwendete Citratplasma muß also nicht tiefgefroren gewesen sein und kann, wenn es tiefgefroren war, rasch auf Temperaturen von + 37 °C aufgetaut und direkt im erfindungsgemäßen Verfahren eingesetzt werden.

Vorzugsweise verwendet man ein Citratplasma, das nach seiner Gewinnung eingefroren wurde und bei 0 bis 8 °C oder auch bei + 37 °C aufgetaut wird. Die gewählte Auftautemperatur entscheidet darüber, ob man in einem Fall größeren Wert auf die bekannte Herstellung von Kryopräzipitat bzw. antihämophilem Globulin A oder aber auf die Herstellung von Fibrinogen legt. Obwohl beide Produkte nebeneinander herstellbar sind, ist die Fibrinogenausbeute aus von Kryopräzipitat befreitem Plasma deutlich geringer als bei der Verarbeitung eines Plasmas, dem das Kryopräzipitat nicht entzogen wurde, da bekanntermaßen Kryopräzipitat sehr viel Fibrinogen enthält. Vor der Adsorption des Plasmas mit kolloidaler Kieselsäure wird das Plasma in bekannter Weise bei pH-Werten von 6,5 bis 8,0 mit ß-Propiolacton und sodann mit UV-Bestrahlung behandelt, um die Hepatitissicherheit der Produkte zu gewährleisten. Das mit kolloidaler Kieselsäure adsorbierte «Citratplasma», aus dem gegebenenfalls die PPSB-Faktoren bereits durch Adsorption an Proteine adsorbierenden Anionenaustauschern entfernt wurden, kann nach einer der Ausführungsformen gemäß Abb. I bis IV weiterverarbeitet werden.

Das erfindungsgemäße Verfahren hat folgende Vorteile gegenüber den bekannten Verfahren:

1. Das als Ausgangsmaterial verwendete Citratplasma fällt üblicherweise bei Blutspenden und Plasmapheresen an und ist daher verhältnismäßig leicht erhältlich.

2. Die sich bezüglich der technischen Verarbeitung ergebenden Probleme der Sterilisation und Pyrogenfreihaltung von Ionenaustauschern auf Basis von Polystyrol, wie sie zur Herstellung von Ionenaustauscherplasma erforderlich sind, sowie deren erhebliche Kosten entfallen.

3. Die Probleme der Ionenaustauscherharze auf Basis von Polystyrol, wie z.B. Abrieb und mögliche Abgabe von niedermolekularen Bestandteilen aus den Harzen, entfallen.

4. Das nach dem erfindungsgemäßen Verfahren erhältliche Fibrinogen ist noch nicht durch Fibrinolyse abgebaut, wie es sonst in Plasmalösungen üblich ist, aus denen das Fibrinogen nicht sofort nach dem Auftauen des Plasmas entfernt wird.

Die Erfindung wird durch die nachstehenden Beispiele näher erläutert:

Beispiel 1

A. Herstellung von antihämophiles Globulin A enthaltendem Konzentrat

Neun Teile Spender-Venenblut wurden zu einem Teil einer 3,8 %igen Natriumcitrat-Stabilisatorlösung gegeben. Das Blut wurde möglichst bald nach der Blutentnahme zentrifugiert und die in physiologischer Kochsalzlösung aufgeschlemmten Erythrozyten dem Spender reinfundiert. Das Plasma wurde spätestens innerhalb von 48 Stunden bei − 40 °C eingefroren.

Das gefrorene Plasma wurde bei einer Temperatur von + 2 bis + 4 °C aufgetaut. Es wurde durch Einzelgefäßzentrifugierung zentrifugiert. Aus der erhaltenen Kälteausfällung (Kryopräzipitat) ließ sich in bekannter Weise ein Konzentrat herstellen, das antihämophiles Globulin A enthielt.

B. Herstellung von hepatitissicherem Fibrinogen

Der in Stufe A beim Zentrifugieren erhaltene kryopräzipitatfreie Citratplasmapool wurde bei Raumtemperatur bis zu einer Konzentration von 0,25 Vol.-% mit frisch destilliertem β-Propiolacton versetzt. Es wurde eine Stunde bei Zimmertemperatur gerührt und während dieser Zeit durch kontinuierliche Zugaben von 1N NaOH-Lösung der pH-Wert bei 7,2 gehalten. Nach UV-Bestrahlung mit einem UV-Durchfluß-Bestrahlungsgerät (Dill-Apparatur) — wurde die Hydrolyse des β-Propiolactons durch kontinuierliche Zugabe von 1N NaOH unter Konstanthaltung des pH-wertes zu Ende geführt, bis eine pH-Konstanz ohne weitere NaOH-Zugabe erreicht war. Das mit β-Propiolacton behandelte und UV-bestrahlte Citratplasma wurde auf 4 °C abgekühlt und bis zu einer Konzentration von 1,5 g kolloidaler Kieselsäure/100 ml Plasma bzw. 250 mg je g Protein mit «Aerosil 380» gemischt. Anschließend wurde zentrifugiert und der Niederschlag zur Fibrinogengewinnung weiterverarbeitet, während der Überstand als Ausgangsmaterial für die Herstellung von PPSB/AT III und der Lösung lagerstabiler Serumproteine verwendet wurde.

Der Aerosil-Niederschlag wurde mit physiologischer Kochsalzlösung gewaschen und anschließend das Fibrinogen mit einer citrathaltigen 15 %igen NaCl-Lösung bei pH 9,5 eluiert. Diese Fibrinogenlösung wurde anschließend zur Entfernung der hohen Kochsalzkonzentration ultrafiltriert. Die ultrafiltrierte Fibrinogenlösung wurde durch eine der Cohn-Fraktionierung analoge Ethanol-Fällung weiter gereinigt. Das mit Ethanol gefällte Fibrinogen wurde in citrathaltiger physiologischer Kochsalzlösung gelöst, anschließend sterilfiltriert und gefriergetrocknet.

C. Herstellung von AT III-haltigem Prothrombinkomplex

10 l fibrinogenfreies Citratplasma, die nach der Arbeitsweise von Stufe B erhalten worden waren, wurden bei Zimmertemperatur ultrafiltriert, wobei (unter Anwendung des Hollowfiber-Systems der Firaa Amicon) gegen 0,45 %ige NaCl-Lösung dialysiert und zunächst das Plasmavolumen auf 2/3 des Ausgangsvolumens ankonzentriert wurde. So wurden die 10 l Plasma in 15 Minuten auf 7 l ankonzentriert und anschließend in 3 Stunden gegen 70 l 0,45 %ige NaCl-Lösung dialysiert. Unter diesen Bedingungen sank die Citratkonzentration von 60 mäq/l unter 1 mäq/l. Anschliessend wurde durch Zugabe von 0,45 %iger NaCl-Lösung das Ausgangsvolumen wieder hergestellt. Dieses von Fibrinogen befreite und ultrafiltrierte «Citratplasma» diente als Ausgangsmaterial für die Herstellung des AT III-haltigen PPSB-Konzentrats.

Durch Zugabe von Tricalciumphosphat bis zu einer Konzentration von 0,8 Gew.-% zu der vorstehenden Proteinlösung und Rühren dieser Mischung bei + 4 °C für 45 Minuten und anschließende Zentrifugation in Bechern wurde das AT III-haltige PPSB durch Elution mit einer 0,05M Trinatriumcitratlösung von dem Tricalciumphosphat-Niederschlag getrennt, während der Überstand des Tricalciumphosphat-Niederschlages als Ausgangsmaterial für die Herstellung der Lösung lagerstabiler Serumproteine diente.

Die AT III-haltige PPSB-Lösung wurde entweder direkt weiter auf ein AT III-haltiges PPSB-Konzentrat aufgearbeitet oder diente als Ausgangsmaterial zur Herstellung von PPSB und einem AT III-Präparat.

Wurde ein AT III-haltiges PPSB-Konzentrat hergestellt, so wurden die Tricalciumphosphat-Eluate mit kolloidaler Kieselsäure (0,5 Gew.-%) adsorbiert und die PPSB/AT III-haltige Lösung nach Entfernung der kolloidalen Kieselsäure durch Zentrifugation mit einem Ultrafiltrationsgerät ankonzentriert bis die Faktor IX-Aktivität der Lösung 30 Einheiten pro ml betrug.

D. Herstellung von AT III und PPSB-Konzentrat

Die in Stufe C erhaltene AT III-haltige PPSB-Lösung wurde gegen 0,9 %ige NaCl-Lösung dialysiert, wobei die Citratkonzentration auf einen Wert von 40 mäq/l sank. Aus dieser Lösung wurden durch Adsorption mit DEAE-Sephadex[R] A 50 (Diethylaminoethyl-Sephadex, Handelsname der Fa. Pharmacia Fine Chemicals für ein mit Epichlorhydrin quervernetztes Dextran) die PPSB-Faktoren adsorbiert und anschließend mit einer 2M NaCl-Lösung, die 0,01M Citrat enthielt, bei pH 7,0 eluiert. Der DEAE-Sephadex A 50-Überstand enthielt das AT III und wurde durch Ankonzentrierung durch Ultrafiltration auf die zehnfache Konzentration eines Normalplasmas gebracht, sterilfiltriert und gefriergetrocknet.

E. Herstellung einer Lösung lagerstabiler Serumproteine

Das in Stufe C als Überstand des Tricalciumphosphat-Niederschlages verbliebene Restplasma wurde 3 Stunden bei 45 °C mit kolloidaler Kieselsäure (Aerosil 380 der Firma Degussa) gemischt, wobei pro 1 l plasma 2 g Aerosil 380 eingesetzt wurden, und gerührt. Anschließend wurde die kolloidale Kieselsäure durch Zentrifugation in Bechern abgetrennt. Die Proteinlösung wurde ultrafiltriert und auf eine Proteinkonzentration von 5 g/100 ml ankonzentriert.

Anschließend wurde diese Proteinlösung über heißluftsterilisierte Membranfilter der Porengröße 0,45 und 0,22 µ in leere sterile Flaschen abgefüllt.

Beispiel 2

Die Arbeitsweise von Beispiel 1 wurde wiederholt mit der Abweichung, daß das Kryopräzipitat nicht aus dem Ausgangsplasma entfernt wurde.

Beispiel 3

Die Arbeitsweise von Beispiel 1 wurde für die Stufen A, B, C und E wiederholt, während in Stufe D bei der Herstellung von AT III und PPSB aus der AT III-haltigen PPSB-Lösung diese zunächst durch Ultrafiltration auf 1/5 ihres Ausgangsvolumens ankonzentriert wurde, bevor die Adsorption des PPSB mit DEAE-Sephadex A 50 erfolgte.

Beispiel 4 (gemäß Abb. I)

Die Arbeitsweise von Beispiel 1 wurde für die Stufen A und B wiederholt.

C. Herstellung von AT III und AT III-haltigem Prothrombinkomplex

10 l fibrinogenfreies Citratplasma, die nach der Arbeitsweise von Stufe B des Beispiels 1 erhalten worden waren, wurden nach der Arbeitsweise von Stufe C des Beispiels 1 ultrafiltriert.

Das so erhaltene ultrafiltrierte Plasma wurde bei einem pH-Wert von 7,2 unter Rühren mit 0,5 g DEAE-Sephadex A 50 pro l Plasma adsorbiert, die vorher wie folgt behandelt worden waren :

150 g DEAE-Sephadex A 50 wurden etwa 20 Studen in 10 l destilliertem Wasser gequollen. Das über dem gequollenen Gel stehende Wasser wurde abgesaugt und durch frisches Wasser ersetzt. Das Gel wurde aufgerührt, und nach dem Absetzen wurden die feinen, noch suspendierten Teilchen abgesaugt. Diese Arbeitsweise wurde noch einmal wiederholt. Anschließend wurde das gequollene und in Wasser suspendierte Gel 20 Minuten bei 121 °C sterilisiert.

Nach der Adsorption am DEAE-Sephadex A 50 wurde das Plasma vom Sephadex abgesaugt und diente als Ausgangsmaterial für die weitere Aufarbeitung auf AT III und die Lösung von Serumproteinen.

Das Sephadex A 50 wurde zweimal mit einer physiologischen Kochsalzlösung gewaschen und dann pro 0,5 g Sephadex A 50 (Trockengewicht) für 20 Minuten bei Zimmertemperatur mit 100 ml eines 0,01M Citratpuffers vom pH-Wert 7,0, der 0,2M an NaCl war, eluiert, der wie folgt erhalten worden war:

11,69 g NaCl und 2,29 g Trinatriumcitrat wurden in etwa 800 ml $H_2O$ gelöst. Der pH-Wert wurde durch Zugabe von 1N HCl auf pH 7,0 eingestellt. Diese Lösung wurde mit $H_2O$ auf 1 l aufgefüllt.

Diese Elution wurde wiederholt. Die vereinigten AT III-Eluate wurden mit einem Ultrafiltrationsgerät (der fa. Amicon) unter Verwendung einer Ultrafiltrationspatrone ankonzentriert.

Anschließend wurden die PPSB-Faktoren vom DEAE-Sephadex A 50 eluiert. Diese Elution erfolgte durch Rühren mit 10 ml eines 0,01M Citratpuffers vom pH-Wert 7,0, der 2M an NaCl war, pro 0,5 g Sephadex A 50 (Trockengewicht) für 20 Minuten bei Zimmertemperatur. Die hierbei verwendete Pufferlösung wurde wie folgt erhalten:

116,9 g NaCl und 2,29 g Trinatriumcitrat wurden in etwa 800 ml $H_2O$ gelöst. Der pH-Wert wurde durch Zugabe von 1N HCl auf pH 7,0 eingestellt. Diese Lösung wurde mit $H_2O$ auf 1 l aufgefüllt.

Die Elution wurde wiederholt. Die Ankonzentrierung der Eluate erfolgte mit einem Ultrafiltrationsgerät (der Fa. Amicon) unter Verwendung einer Ultrafiltrationspatrone (Amicon SM 10). Das Retentat aus der Ankonzentrierung wurde mit der Gerinnungsaktivitätsmessung auf die Aktivitäten der PPSB-Faktoren überprüft. Anschließend wurde das PPSB-Konzentrat mit 5 Einheiten Heparin/ml versetzt und sterilfiltriert.

Beim Lösen des so erhaltenen PPSB-Konzentrates nach der Trocknung in destilliertem Wasser ergaben sich die in der nachstehenden Tabelle VI aufgeführten Eigenschaften.

Tabelle VI
Eigenschaften des PPSB-Konzentrates

| Protein g/100 ml | Na$^+$ mäq/l | Cl$^-$ mäq/l | Citrat mäq/l | Ca$^{++}$ mäq/l | Aktivität in % d.N. | | | |
|---|---|---|---|---|---|---|---|---|
| | | | | | II | VII | IX | X |
| etwa 2,5 | 140 | 125 | 110 | 45 | 2 300 | 1 800 | 2 500 | 2 500 |

Bei den Angaben der Tabelle VI ist zu berücksichtigen, daß das Volumen des gelösten PPSB-Präparates um etwa 10 % größer als das abgefüllte Volumen war (Volumenvergrößerung durch die Trockensubstanz).

Das PPSB-Konzentrat hatte einen AT III-Gehalt, der etwa 2 bis 4 mal so hoch wie der eines Normalplasmas war. Die TGt$_{50}$ eines solchen Präparates betrung mehrere Stunden.

D. Herstellung von AT III

a) durch Adsorption an Tricalciumphosphat

Das in Stufe C nach der Adsorption am DEAE-Sephadex A 50 vom Sephadex abgesaugte Plasma wurde mit 1N NaOH auf einen pH-Wert von 8,0 gebracht und mit 0,8 Gew.-% Tricalciumphosphat ($Ca_5(PO_4)OH$, Pentacalciumhydroxytriphosphat, Molekulargewicht 502, 32, Art.-Nr. 2 143 von Merck) versetzt. Das Tricalciumphosphat wurde vor Gebrauch 2 Stunden auf 180 °C erhitzt. Die Adsorptionszeit betrug 40 Minuten. Die anschließende Trennung des Tricalciumphosphates vom Plasma erfolgte durch Zentrifugation mit der Durchflußzentrifuge.

Der Plasmaüberstand wurde als Ausgangsmaterial für Stufe E verwendet.

Um das Antithrombin III vom Tricalciumphosphat-Niederschlag zu eluieren, wurde dieser Niederschlag zunächst für die Dauer von 10 Minuten durch Anrühren mit etwa 33 Vol.-% an 0,9 %iger NaCl-Lösung, bezogen auf das Plasmaausgangsvolumen, gewaschen. Anschließend wurde zentrifugiert. Anschließend wurde das AT III 30 minuten bei Zimmertemperatur unter Rühren mit 5 Vol.-%, bezogen auf das ultrafiltrierte Plasma, an 0,05M Trinatriumcitrat-2-hydrat (Merck) bei einem pH-Wert von 8,8 bis 9,1 eluiert. Dann wurde das Tricalciumphosphat von der AT III-Lösung durch Zentrifugation getrennt. Eine zweite Elution des Tricalciumphosphat-Niederschlages wurde analog zur ersten Elution durchgeführt. Das verwendete Volumen des Elutionsmittels betrug 2,5 Vol.-%, bezogen auf das ultrafiltrierte Plasma.

Anschließend wurde bei Zimmertemperatur eine Ankonzentrierung der AT III-Lösung durch Ultrafiltration mit einem Ultrafiltrationsgerät (der Fa. Amicon) unter Verwendung einer Ultrafiltrationspatrone (Amicon PM 10) durchgeführt. Das Retentat aus der Ankonzentrierung der Lösung ergab eine etwa 10-fache AT III-Anreicherung gegenüber Normalplasma.

Beim Lösen des erhaltenen, getrockneten AT III-Konzentrates in destilliertem Wasser wurden die in Tabelle VII aufgeführten Eingenschaften festgestellt.

Tabelle VII
Eigenschaften des AT III Konzentrates

| Protein g/100 ml | Na$^+$ mäq/l | Cl$^-$ mäq/l | Citrat mäq/l | Ca$^{++}$ mäq/l | AT III in IU/ml |
|---|---|---|---|---|---|
| etwa 4,0-5,0 | 140 | 25 | 110 | 45 | 200 |

Bei den Angaben in Tabelle VII ist zu berücksichtigen, daß das Volumen des gelösten AT III-Präparates um etwa 10 % größer als das abgefüllte Volumen ist (Volumenvergrößerung durch die Trockensubstanz).
b) Herstellung von AT III durch Adsorption an DEAE-Sephadex A 50 oder DEAE-Cellulose
Die in Stufe D a) beschriebene Arbeitsweise wurde wiederholt, wobei jedoch als Adsorbens anstelle von Tricalciumphosphat DEAE-Sephadex A 50 oder DEAE-Cellulose verwendet wurden.
Es wurde ein analoges Antithrombin III-Konzentrat erhalten.

E. Herstellung einer Lösung lagerstabiler Serumproteine

Das in Stufe D a) als Überstand des Tricalciumphosphat-Niederschlages verbliebene Plasma wurde 3 Stunden bei + 45 °C mit 2 % Aerosil AE 380 adsorbiert und anschliessend zur Abtrennung des Aerosils zentrifugiert. Zur Klärung dieser Proteinlösung wurde sie über heißluftsterilisierte Membranen einer Porengröße von 5,0 μ gegeben.
Um den Gehalt der nach der Aerosil-Adsorption geklärten Lösung an Silikat, Calcium und Phosphat zu verringern, wurde sie unter Verwendung von Ultrafiltrationspatronen (Amicon PM 10) bei etwa + 20 °C ultrafiltriert. Die ultrafiltrierte Proteinlösung wurde sodann über heißluftsterilisierte Membranfilter der Porengröße 0,45 und 0,22 μ in leere, sterile 1 l-Flaschen abgefüllt.
Das in Stufe D b) als Überstand des Niederschlages aus DEAE-Sephadex A 50 verbliebene Plasma wurde auf gleiche Weise wie vorstehend beschrieben an Aerosil AE 380 adsorbiert, ultrafiltriert und sterilfiltriert, um daraus die Lösung der lagerstabilen Serumproteine zu erhalten.

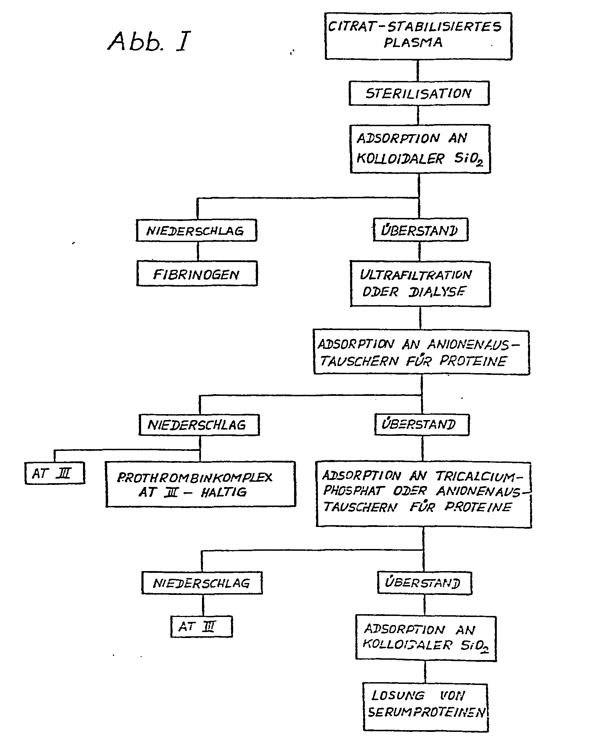

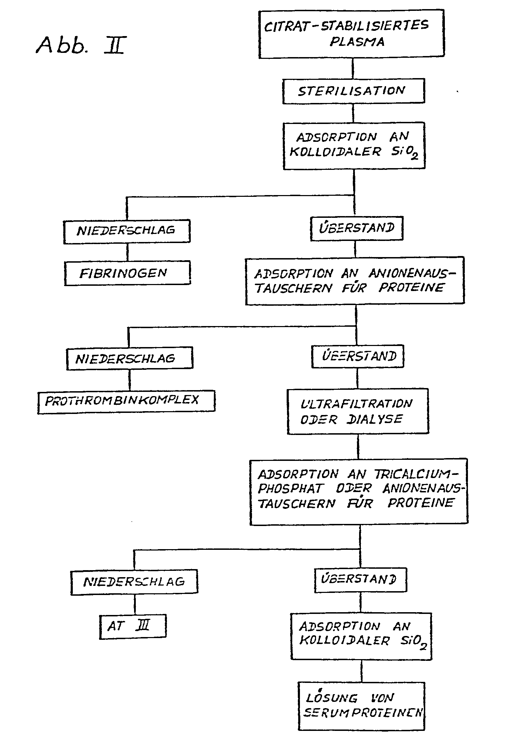

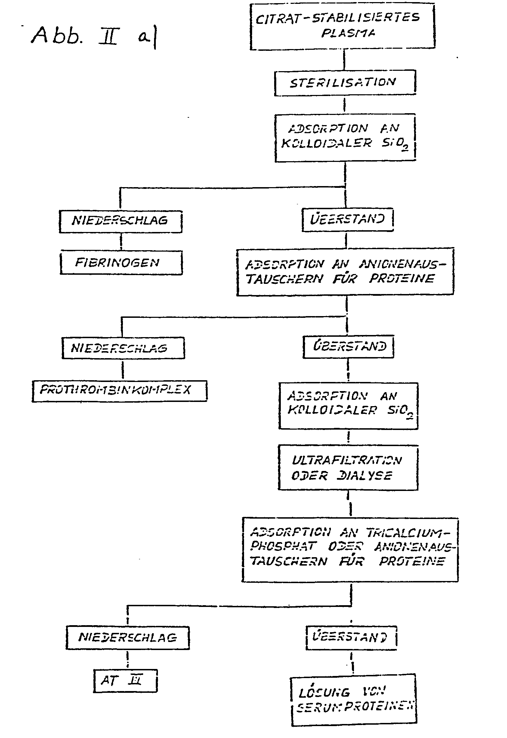

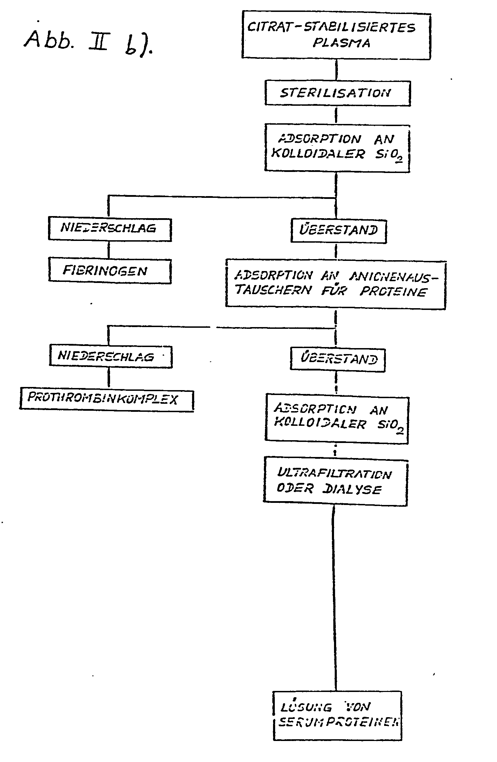

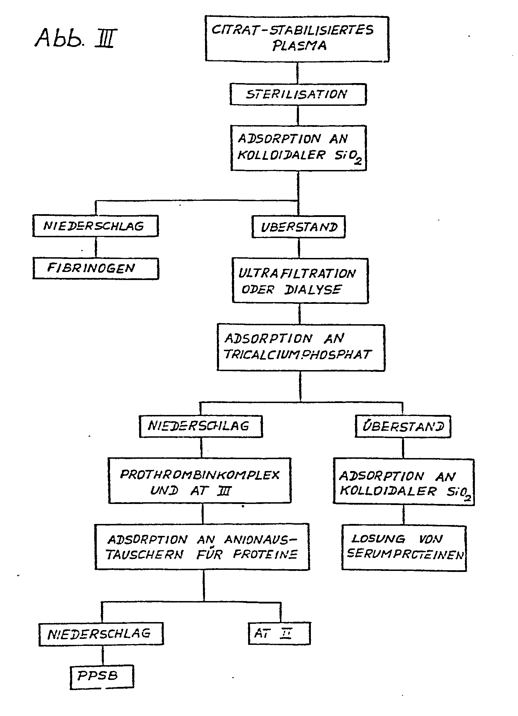

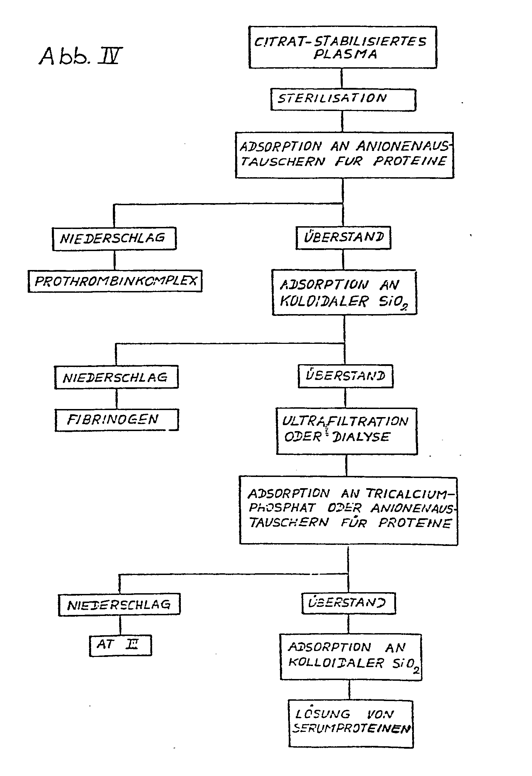

**Ansprüche**

1. Verfahren zur Herstellung von Fibrinogen, einem die Gerinnungsfaktoren II, VII, IX und X enthaltenden Prothrombinkomplex, der Antithrombin III enthalten kann, Antithrombin III und einer Lösung von lagerstabilen Serumproteinen aus einem mit Citrat stabilisierten Blutplasma, dadurch gekennzeichnet, daß man aus dem Plasma durch Adsorption an kolloidaler Kieselsäure mit einer spezifischen Oberfläche von 50 bis 400 m$^2$/g, die in einer Konzentration von 50 bis 400 mg je g Plasmaprotein angewendet wird, Fibrinogen isoliert ; anschließend entweder a) zunächst durch Ultrafiltration oder Dialyse Citrat- und Calciumionen entfernt und dann aus der Proteinlösung über Proteine adsorbierenden Anionenaustauschern oder Tricalciumphosphat die Gerinnungsfaktoren II, VII, IX und X und Antithrombin III adsorbiert oder b) die Gerinnungsfaktoren II, VII, IX und X vor der Ultrafiltration oder Dialyse adsorbiert, wobei Antithrombin III nicht gleichzeitig adsorbiert wird, und Antithrombin III erst nach der Entfernung der Citrat- und Calciumionen durch Ultrafiltration oder Dialyse an den genannten Adsorbentien adsorbiert ; und sodann aus der verbleibenden Plasmaflüssigkeit weitere unstabile Proteine durch eine erneute Adsorption an kolloidaler Kieselsäure entfernt und eine Lösung von lagerstabilen Serumproteinen gewinnt.
2. Verfahren nach Anspruch 1, dadurch gekennzeichnet, daß man zur Adsorption von Antithrombin III und den Gerinnungsfaktoren II, VII, IX und X aus dem mit kolloidaler Kieselsäure adsorbierten und ultrafiltrierten oder dialysierten Citratplasma Proteine adsorbierende Anionenaustauscher, insbesondere Diethylaminoethylgruppen tragende, quervernetzte Dextrane oder Cellulose, verwendet.
3. Verfahren nach Anspruch 1, dadurch gekennzeichnet, daß man zur Adsorption von Antithrombin III und den Gerinnungsfaktoren II, VII, IX und X aus dem mit kolloidaler Kieselsäure adsorbierten und ultrafiltrierten oder dialysierten Citratplasma handelsübliches Tricalciumphosphat verwendet.
4. Verfahren nach Anspruch 2 oder 3, dadurch gekennzeichnet, daß man Antithrombin III von den Adsorbentien mit einem Citrat/NaCl-Puffer niedriger Molarität eluiert und anschließend die Gerinnungsfaktoren II, VII, IX und X mit einem Citrat/NaCl-Puffer höherer Molarität eluiert.
5. Verfahren nach einem der Ansprüche 1 bis 4, dadurch gekennzeichnet, daß man durch eine zweite Adsorption an Proteine adsorbierenden Anionenaustauschern oder Tricalciumphosphat weiteres Antithrombin III adsorbiert und durch Elution von diesen Adsorbentien gewinnt.
6. Verfahren nach Anspruch 1, dadurch gekennzeichnet, daß man zur Adsorption der Gerinnungsfaktoren II, VII, IX und X aus dem mit kolloidaler Kieselsäure adsorbierten Citratplasma Proteine adsorbierende Anionenaustauscher, insbesondere Diethylaminoethylgruppen tragende, quervernetzte Dextrane oder Cellulose, verwendet, anschließend das adsorbierte Plasma ultrafiltriert oder dialysiert und dann Antithrombin III an den vorstehend genannten Adsorbentien adsorbiert.

7. Verfahren nach einem der Ansprüche 1 bis 6, dadurch gekennzeichnet, daß man das an kolloidaler Kieselsäure adsorbierte Fibrinogen mit 5 bis 20 % Alkalihalogenide enthaltenden Pufferlösungen eluiert und in bekannter Weise zu einem für therapeutische Zwecke geeigneten Fibrinogenpräparat aufarbeitet.

8. Verfahren nach einem der Ansprüche 1 bis 7, dadurch gekennzeichnet, daß man als Ausgangsmaterial ein mit ß-Propiolacton und UV-Bestrahlung behandeltes Citratplasma verwendet.

9. Verfahren nach einem der Ansprüche 1 bis 8, dadurch gekennzeichnet, daß man zur Adsorption des Fibrinogens die kolloidale Kieselsäure in einer Konzentration von 150 bis 250 mg je g Plasmaprotein verwendet.

10. Verfahren nach Anspruch 1, dadurch gekennzeichnet, daß man vor der Adsorption über kolloidaler Kieselsäure eine Adsorption der Gerinnungsfaktoren II, VII, IX und X an Proteine adsorbierenden Anionenaustauschern durchführt.

## Claims

1. Process for preparing fibrinogen, a prothrombin complex containing the coagulation factors II, VII, IX and X that can contain antithrombin III, antithrombin III and a solution of storage-stable serum proteins from a blood plasma stabilized with citrate, characterized in that from the plasma, by adsorption on colloidal silica of a specific surface of 50 to 400 m²/g, which is employed in a concentration of from 50 to 400 mg per g of plasma protein, fibrinogen is isolated ; and in that thereupon either a) at first citrate and calcium ions are removed by ultrafiltration of dialysis and then from the protein solution, over anion exchangers that adsorb proteins, or tricalcium phosphate, the coagulation factors II, VII, IX and X and antithrombin III are adsorbed, or b) the coagulation factors II, VII, IX and X are adsorbed prior to the ultrafiltration of dialysis, antithrombin III not being simultaneously adsorbed, and antithrombin III is adsorbed on the said adsorbents only after the removal of the citrate and calcium ions by ultrafiltration or dialysis ; and in that thereupon from the remaining plasma fluid further unstable proteins are removed by another adsorption on colloidal silica and a solution of storage-stable serum proteins is obtained.

2. Process according to claim 1, characterized in that anion exchangers that adsorb proteins, in particular diethylaminoethyl groups carrying crosslinked dextrans or cellulose, are employed for the adsorption of antithrombin III and the coagulation factors II, VII, IX and X from the citrate plasma adsorbed with colloidal silica and ultrafiltered or dialyzed.

3. Process according to claim 1, characterized in that commercial tricalcium phosphate is employed for adsorbing antithrombin III and the coagulation factors II, VII, IV and X from the citrate plasma adsorbed with colloidal silica and ultrafiltered or dialyzed.

4. Process according to claims 2 or 3, characterized by eluting antithrombin III from the adsorbents with a citrate/NaCl butter of low molarity, and subsequently eluting the coagulation factors II, VII, IX and X with a citrate/NaCl buffer of higher molarity.

5. Process according to one of claims 1 to 4, characterized in that additional antithrombin III is adsorbed by a second adsorption on anion exchangers that adsorb proteins, or tricalcium phosphate and is obtained by eluting said adsorbents.

6. Process according to claim 1, characterized by employing anion exchangers that adsorb proteins, in particular diethylaminoethyl groups carrying cross-linked dextrans or cellulose, for the adsorption of the coagulation factors II, VII, IX and X from the citrate plasma adsorbed with colloidal silica, subsequently ultrafiltering or dialyzing the adsorbed plasma and then adsorbing antithrombin III on the said adsorbents.

7. Process according to one of claims 1 to 6, characterized by eluting the fibrinogen adsorbed on colloidal silica with buffer solutions containing of from 5 to 20 % alkali halides and processing it in a manner known per se to a fibrinogen preparation suitable for therapeutical purposes.

8. Process according to one of claim 1 to 7, characterized in that as starting material a citrate plasma treated with ß-propiolactone and UV radiation is employed.

9. Process according to one of claims 1 to 8, characterized in that for the adsorption of the fibrinogen the colloidal silica is employed in a concentration of from 150 to 250 mg per g of plasma protein.

10. Process according to claim 1, characterized by conducting an adsorption of the coagulation factors II, VII, IX and X on anion exchangers that adsorb proteins prior to the adsorption on colloidal silica.

## Revendications

1. Procédé de préparation du fibrinogène, d'un complexe de prothrombine contenant les facteurs de coagulation II, VII, IX et X, qui peut contenir de l'antithrombine III, de l'antithrombine et d'une solution de protéines sériques stables au stockage à partir d'un plasma sanguin stabilisé au citrate, caractérisé en ce qu'on isole le fibrinogène du plasma par adsorption sur de l'acide silicique colloïdal ayant une surface spécifique de 50 à 400 m²/g, utilisé à une concentration de 50 à 400 mg/g de protéine du plasma ; puis en ce que a) on élimine d'abord les ions citrate et calcium par ultrafiltration ou dialyse, puis on adsorbe sur des échangeurs d'anions adsorbant les protéines ou sur du phosphate tricalcique les facteurs de coagulation II, VII, IX et X et l'antithrombine III ou b) on adsorbe les facteurs de coagulation II, VII, IX et X avant l'ultrafiltration ou la dialyse, l'antithrombine III n'étant pas adsorbée simultanément, et on adsorbe l'antithrombine III seulement après élimination des ions citrate et calcium par ultrafiltration ou dialyse sur les adsorbants précités ; puis on élimine du liquide plasmatique restant d'autres protéines instables par une nouvelle adsorption sur de l'acide silicique colloïdal, et l'on obtient une solution de protéines du sérum stable au stockage.

2. Procédé selon la revendication 1, caractérisé en ce qu'on utilise pour l'adsorption de l'antithrombine III et des facteurs de coagulation II, VII, IX et X à partir du plasma citraté adsorbé sur acide silicique colloïdal et ultrafiltré ou dialysé, des échangeurs d'anions adsorbant les protéines, en particulier du dextrane ou de la cellulose portant des groupements éthylaminoéthyle, réticulé.

3. Procédé selon la revendication 1, caractérisé en ce qu'on utilise pour l'adsorption de l'antithrombine III et des facteurs de coagulation II, VII, IX et X, à partir de plasma citraté adsorbé sur acide silicique colloïdal et ultrafiltré ou dialysé, du phosphate tricalcique du commerce.

4. Procédé selon l'une quelconque des revendications 2 ou 3, caractérisé en ce qu'on élue l'antithrombine III des adsorbants avec un tampon citrate/NaCl de faible molarité puis en ce qu'on élue les facteurs de coagulation II, VII, IX et X avec un tampon citrate/NaCl de molarité supérieure.

5. Procédé selon l'une quelconque des revendications 1 à 4, caractérisé en ce qu'on adsorbe un supplément d'antithrombine III par une deuxième adsorption sur des échangeurs d'anions adsorbant les protéines ou sur du phosphate tricalcique et en ce qu'on l'obtient par élution de ces adsorbants.

6. Procédé selon la revendication 1, caractérisé en ce qu'on utilise pour l'adsorption des facteurs de coagulation II, VII, IX et X à partir du plasma citraté adsorbé sur acide silicique colloïdal, des échangeurs d'anions adsorbant les protéines, en particulier des dextranes portant des groupements diéthylaminoéthyle, réticulés ou de la cellulose, puis en ce qu'on soumet à une ultrafiltration le plasma adsorbé et en ce qu'on adsorbe l'antithrombine III sur les adsorbants précités.

7. Procédé selon l'une quelconque des revendications 1 à 6, caractérisé en ce qu'on élue le fibrinogène adsorbé sur l'acide silicique colloïdal avec des solutions tampons contenant 5 à 20 % d'halogénures alcalins et en ce qu'on poursuit le traitement d'une manière connue pour obtenir une préparation de fibrinogène convenant à des fins thérapeutiques.

8. Procédé selon l'une quelconque des revendications 1 à 7, caractérisé en ce qu'on utilise comme matière de départ un plasma citraté traité par la ß-propiolactone et par irradiation UV.

9. Procédé selon l'une quelconque des revendications 1 à 8, caractérisé en ce qu'on utilise pour l'adsorption du fibrinogène l'acide silicique colloïdal à une concentration de 150 à 250 mg par gramme de protéines du plasma.

10. Procédé selon la revendication 1, caractérisé en ce qu'avant l'adsorption sur acide silicique, on effectue une adsorption des facteurs de coagulation II, VII, IX et X sur des échangeurs d'anions adsorbant les protéines.

- 1/6 -

*Abb. I*

Abb. II

```
                              ┌─────────────────────┐
                              │ CITRAT-STABILISIERTES│
                              │       PLASMA         │
                              └──────────┬──────────┘
                                         │
                              ┌──────────┴──────────┐
                              │   STERILISATION      │
                              └──────────┬──────────┘
                                         │
                              ┌──────────┴──────────┐
                              │   ADSORPTION  AN     │
                              │  KOLLOIDALER SiO₂   │
                              └──────────┬──────────┘
```

$$\text{ADSORPTION AN KOLLOIDALER } SiO_2$$

| NIEDERSCHLAG | | ÜBERSTAND |
|---|---|---|

| FIBRINOGEN | | ADSORPTION AN ANIONENAUS-TAUSCHERN FÜR PROTEINE |
|---|---|---|

| NIEDERSCHLAG | | ÜBERSTAND |
|---|---|---|

| PROTHROMBINKOMPLEX | | ULTRAFILTRATION ODER DIALYSE |
|---|---|---|

ADSORPTION AN TRICALCIUM-PHOSPHAT ODER ANIONENAUS-TAUSCHERN FÜR PROTEINE

| NIEDERSCHLAG | | ÜBERSTAND |
|---|---|---|

| AT III | | ADSORPTION AN KOLLOIDALER SiO₂ |
|---|---|---|

LÖSUNG VON SERUMPROTEINEN

- 3/6 -

Abb. II a)

```
        ┌──────────────────────────┐
        │  CITRAT-STABILISIERTES   │
        │        PLASMA            │
        └──────────────────────────┘
                    │
        ┌──────────────────────────┐
        │     STERILISATION        │
        └──────────────────────────┘
                    │
        ┌──────────────────────────┐
        │    ADSORPTION AN         │
        │  KOLLOIDALER SiO₂        │
        └──────────────────────────┘
                    │
          ┌─────────┴──────────┐
          │                    │
┌──────────────────┐   ┌──────────────┐
│  NIEDERSCHLAG    │   │  ÜBERSTAND   │
└──────────────────┘   └──────────────┘
          │                    │
┌──────────────────┐   ┌────────────────────────────┐
│   FIBRINOGEN     │   │  ADSORPTION AN ANIONENAUS-  │
└──────────────────┘   │  TAUSCHERN FÜR PROTEINE     │
                       └────────────────────────────┘
                                │
                    ┌───────────┴────────────┐
                    │                         │
        ┌──────────────────┐        ┌──────────────┐
        │  NIEDERSCHLAG    │        │  ÜBERSTAND   │
        └──────────────────┘        └──────────────┘
                    │                         │
    ┌──────────────────────────┐    ┌──────────────────┐
    │  PROTHROMBINKOMPLEX      │    │  ADSORPTION AN   │
    └──────────────────────────┘    │  KOLLOIDALER SiO₂│
                                    └──────────────────┘
                                            │
                                    ┌──────────────────┐
                                    │  ULTRAFILTRATION │
                                    │  ODER DIALYSE    │
                                    └──────────────────┘
                                            │
                            ┌────────────────────────────────┐
                            │  ADSORPTION AN TRICALCIUM-     │
                            │  PHOSPHAT ODER ANIONENAUS-     │
                            │  TAUSCHERN FÜR PROTEINE        │
                            └────────────────────────────────┘
                                        │
                            ┌───────────┴────────────┐
                            │                         │
                ┌──────────────────┐        ┌──────────────┐
                │  NIEDERSCHLAG    │        │  ÜBERSTAND   │
                └──────────────────┘        └──────────────┘
                            │                         │
                    ┌──────────┐          ┌──────────────────┐
                    │  AT III  │          │  LÖSUNG VON      │
                    └──────────┘          │  SERUMPROTEINEN  │
                                          └──────────────────┘
```

- 4/6 -

Abb. II b).

```
                              ┌────────────────────────┐
                              │ CITRAT-STABILISIERTES  │
                              │        PLASMA          │
                              └────────────────────────┘
                                        │
                              ┌────────────────────────┐
                              │     STERILISATION      │
                              └────────────────────────┘
                                        │
                              ┌────────────────────────┐
                              │    ADSORPTION  AN       │
                              │   KOLLOIDALER  SiO₂     │
                              └────────────────────────┘
                                        │
                    ┌───────────────────┴──────────────┐
          ┌──────────────────┐              ┌──────────────────┐
          │   NIEDERSCHLAG    │              │    ÜBERSTAND      │
          └──────────────────┘              └──────────────────┘
                    │                                  │
          ┌──────────────────┐         ┌──────────────────────────────┐
          │    FIBRINOGEN     │         │  ADSORPTION AN ANIONENAUS-   │
          └──────────────────┘         │   TAUSCHERN FÜR PROTEINE      │
                                        └──────────────────────────────┘
                                                   │
                          ┌────────────────────────┴────────┐
                ┌──────────────────┐              ┌──────────────────┐
                │  NIEDERSCHLAG     │              │    ÜBERSTAND      │
                └──────────────────┘              └──────────────────┘
                          │                                  │
          ┌──────────────────────────┐          ┌──────────────────┐
          │  PROTHROMBINKOMPLEX       │          │   ADSORPTION AN   │
          └──────────────────────────┘          │  KOLLOIDALER SiO₂ │
                                                 └──────────────────┘
                                                          │
                                                 ┌──────────────────┐
                                                 │  ULTRAFILTRATION  │
                                                 │   ODER DIALYSE    │
                                                 └──────────────────┘
                                                          │
                                                 ┌──────────────────┐
                                                 │  LÖSUNG VON       │
                                                 │  SERUMPROTEINEN   │
                                                 └──────────────────┘
```

- 5/6 -

Abb. III

CITRAT-STABILISIERTES PLASMA

STERILISATION

ADSORPTION AN KOLLOIDALER $SiO_2$

NIEDERSCHLAG

FIBRINOGEN

UBERSTAND

ULTRAFILTRATION ODER DIALYSE

ADSORPTION AN TRICALCIUM.PHOSPHAT

NIEDERSCHLAG

PROTHROMBINKOMPLEX UND AT III

ADSORPTION AN ANIONAUS-TAUSCHERN FÜR PROTEINE

NIEDERSCHLAG

AT III

PPSB

UBERSTAND

ADSORPTION AN KOLLOIDALER $SiO_2$

LOSUNG VON SERUMPROTEINEN

- 6/6 -

Abb. IV

CITRAT-STABILISIERTES PLASMA

↓

STERILISATION

↓

ADSORPTION AN ANIONENAUS-TAUSCHERN FUR PROTEINE

├─ NIEDERSCHLAG
│     ↓
│  PROTHROMBINKOMPLEX
│
└─ ÜBERSTAND
      ↓
   ADSORPTION AN KOLOIDALER $SiO_2$

├─ NIEDERSCHLAG
│     ↓
│  FIBRINOGEN
│
└─ ÜBERSTAND
      ↓
   ULTRAFILTRATION ODER DIALYSE

↓

ADSORPTION AN TRICALCIUM-PHOSPHAT ODER ANIONENAUS-TAUSCHERN FÜR PROTEINE

├─ NIEDERSCHLAG
│     ↓
│  AT III
│
└─ ÜBERSTAND
      ↓
   ADSORPTION AN KOLLOIDALER $SiO_2$
      ↓
   LÖSUNG VON SERUMPROTEINEN